# EUROPÄISCHE PATENTSCHRIFT

(11) **EP 1 154 776 B1**
(45) Veröffentlichungstag und Bekanntmachung des Hinweises auf die Patenterteilung: **17.08.2005**
(21) Anmeldenummer: 00907514.4
(22) Anmeldetag: 08.02.2000
(51) Int. Cl.: A61K 31/519, A61P 25/32

(54) **PHARMAZEUTISCHE ZUSAMMENSETZUNG, ENTHALTEND DESOXYPEGANIN ZUR BEHANDLUNG DES ALKOHOLISMUS**
PHARMACEUTICAL COMPOSITION CONTAINING DESOXYPEGANINE FOR THE TREATMENT OF ALCOHOLISM
COMPOSITION PHARMACEUTIQUE CONTENANT DE LA DESOXYPEGANINE UTILISEE POUR LE TRAITEMENT DE L'ALCOOLISME

(30) Priorität: 19.02.1999 DE 19906974
(43) Veröffentlichungstag der Anmeldung: 21.11.2001
(73) Patentinhaber: HF Arzneimittelforschung GmbH, 59368 Werne (DE)
(72) Erfinder: ASMUSSEN, Bodo, D-56170 Bendorf (DE); HILLE, Thomas, D-56567 Neuwied (DE); HOFFMANN, Hans-Rainer, D-56566 Neuwied (DE); OPITZ, Klaus, D-48157 Münster (DE)
(74) Vertreter: Flaccus, Rolf-Dieter, Dr.
(86) Internationale Anmeldenummer: PCT/EP2000/000973
(87) Internationale Veröffentlichungsnummer: WO 2000/048600

(56) Entgegenhaltungen:
- DE-A- 19 509 663
- FR-A- 2 668 062
- DATABASE WPI Section Ch, Week 197913 Derwent Publications Ltd., London, GB; Class B02, AN 1979-25213B XP002139611 & SU 605 614 A (AS UZB CHEM GROWING), 6. April 1978 (1978-04-06)
- DATABASE WPI Section Ch, Week 198235 Derwent Publications Ltd., London, GB; Class B04, AN 1982-74317E XP002139612 & SU 878 295 A (AS UZB VEGET CHEM), 7. November 1981 (1981-11-07)

## Beschreibung

Desoxypeganin und seine pharmazeutisch annehmbaren Säureadditionssalze können zur Behandlung des Alkoholismus verwendet werden. Diese Substanzen werden vorzugsweise in einer kontinuierlichen und kontrollierten Weise verabreicht. Die pharmazeutische Darreichungsform ermöglicht eine kontrollierte Freisetzung für z. B. orale, transdermale oder auf anderem Wege parenterale Verabreichung.

Die Erfindung betrifft die Verwendung von Desoxypeganin sowie seiner pharmazeutisch geeigneten Säureadditionssalze zur Behandlung des Alkoholismus. Diese Verbindungen werden aus entsprechenden pharmazeutischen Formulierungen, die z. B. oral, transdermal oder anderweitig parenteral verabreicht werden, auf kontinuierliche und kontrollierte Weise freigesetzt.

Insbesondere stellt die vorliegende Erfindung pharmazeutische Formulierungen zur Verfügung, die geeignete Verbindungen zur Behandlung des Alkoholismus in kontrollierter Weise freisetzen.

Während der akute Entzug bei Alkoholabhängigen und die Behandlung des lebensgefährlichen Entzugdelirs in Spezialabteilungen heute kein medizinisches Problem mehr darstellen, gibt es noch immer keine befriedigende Behandlung des chronischen Alkoholismus. Etwa 80% der therapierten Alkoholiker werden innerhalb eines Jahres rückfällig. Sie bedürfen der Hilfe durch ein zuverlässig wirkendes und gut verträgliches Mittel gegen das zum Rückfall führende Alkoholverlangen.

Trotz der mit dem Problem des chronischen Alkoholismus verbundenen menschlichen Probleme und des immensen volkswirtschaftlichen Schadens stagniert die Innovation der medikamentösen Behandlung des chronischen Alkoholismus weltweit und ganz besonders in der Bundesrepublik Deutschland.

Bei den Pharmaka, die zur Bekämpfung der Alkoholwirkungen und des Alkoholismus eingesetzt werden, müssen unterschieden werden:
1. Ernüchterungsmittel (Amethystika).
2. Mittel zur Behandlung der Iebensbedrohenden Alkoholvergiftung, z. B. Naloxon (Narcanti), Physostigmin.
3. Pharmaka zur Erleichterung des akuten Alkoholentzugs, z. B. Neuroleptika (Neurocil, Melleril), Piracetam, Clonidin, Carbamazepin, sowie des Entzugsdelirs: Clomethiazol (Distraneurin).
4. Substanzen, die den Alkoholabbau auf der Stufe des Acetaldehyds blockieren und dadurch eine künstliche Alkoholunverträglichkeit erzeugen.
   Disulfiram (Antabus), Hauptvertreter dieser Gruppe, ist das Medikament, das am häufigsten zur Behandlung der Alkoholkrankheit eingesetzt wird. Antabus ist jedoch kein Heilmittel, da diese und verwandte Substanzen weder das Alkoholverlangen mindern noch die Ursachen der Erkrankung beeinflussen.
5. Mittel, die das zwanghafte Alkoholverlangen (das "craving") stillen und so dem Rückfälligwerden der therapierten Alkoholiker vorbeugen sollen. Insbesondere an diesen Mitteln besteht ein dringender Bedarf, ein wirksames Medikament zur Behandlung des chronischen Alkoholismus ist jedoch noch nicht gefunden worden.

Klinische Versuche mit Fenfluramin und Bromocriptin sind nicht wiederholt und bestätigt worden.

Die grossen Hoffnungen, die man in die Lithiumtherapie des chronischen Alkoholismus gesetzt hatte, haben sich nicht erfüllt.

Die besten Erfolge sind noch mit Zimelidin erzielt worden, aber dieser Serotonin-Wiederaufnahmehemmer, der unter dem Namen Normud als Antidepressivum im Handel war, musste wegen schwerwiegender Nebenwirkungen zurückgezogen werden und steht nicht mehr zur Verfügung. Galanthamin, ein Alkaloid aus Amaryllidaceen wird z. Zt. noch klinisch getestet.

DE 195 09 663 offenbart ein Verfahren zur Isolierung des Alkaloids Galanthamin, das nach diesem Verfahren hergestellte Galanthamin selbst, die Verwendung des so hergestellten Galanthamins in galenischen Zubereitungsformen, sowie das so hergestellte Galanthamin zur Behandlung des Engwinkelglaukoms, der Alzheimerschen Krankheit und der Alkohol- und Nikotinabhängigkeit.

Aufgabe der Erfindung ist daher die Bereitstellung eines Arzneistoffs, der durch kontrollierte Abgabe aus einer oralen, transdermalen oder anderweitig parenteralen Formulierung eine wirksame und praktische Behandlung des Alkoholismus ermöglicht, indem er das Verlangen nach Alkohol mindert.

Diese Aufgabe wird erfindungsgemäss gelöst durch eine Formulierung und ihre Verwendung zur Behandlung des Alkoholismus, die dadurch gekennzeichnet ist, dass sie eine wirksame Menge des Wirkstoffs Desoxypeganin (1,2,3,9-Tetrahydropyrrolo[2,1-b]chinazolin) und / oder eines seiner pharmazeutisch verträglichen Säureadditionssalze enthält.

Diese Lösung ist umso überraschender als die pharmakologischen Wirkungen des Desoxypeganins in der ehemaligen UdSSR zwar intensiv untersucht worden sind, die auf Desoxypeganin zurückgehende Wirkung einer desoxypeganinhaltigen Formulierung, das Verlangen Alkoholkranker nach Alkohol zu mindern, aber bisher nicht beschrieben wurde.

SU 605 614 A beschreibt die Eigenschaft von Desoxypeganin als Anticholinesterase-Wirkstoff zu wirken. Desoxypeganin wird als neuer Arzneistoff beschrieben, der die Aktivität von Acetylcholin auf den arteriellen Druck, sowie die Herzfunktion stimuliert. Weiterhin sind diverse Indikationen angegeben, z.B. Myasthenie, Myopathie und motorische, sowie sensorische Störungen. Gegenstand des Dokuments ist weiterhin die Herstellung des Hydrochlorids aus der Base, d.h. der Form, in der das Alkaloid Desoxypeganin aus der Pflanze Peganum Harmala gewonnen werden kann.

SU 878 295 B beschreibt ein Verfahren zur Extraktion von Desoxypeganin aus dem grünen Teil der Pflanze Peganum Harmala. Dabei wird das Hydrochlorid hergestellt, welches durch Umkristalisation gereinigt werden kann.

Aufgrund seiner pharmakologischen Eigenschaften gehört Desoxypeganin zur Gruppe der reversibel wirkenden Cholinesterasehemmstoffe. Es steht in seinen Wirkungen dem Physostigmin, dem Neostigmin und dem Galanthamin nahe, besitzt jedoch auch spezifische Eigenschaften. Desoxypeganin hemmt nicht nur den Abbau von Acetylcholin, sondern auch den von Dopamin.

Dieser Vorteil wiegt seine etwas geringere dosisbezogene Cholinesterasehemmwirkung auf. Desoxypeganin war in der ehemaligen Sowjetunion als Antidot und Prophylaktikum bei Vergiftungen durch organische Thiophosphorsäureester vorgesehen.

Im Gegensatz zu Neostigmin überwindet Desoxypeganin die Bluthirnschranke und antagonisiert die cerebrale Wirkung cholinerger Gifte.

Die vorliegende Erfindung ist auf Formulierungen gerichtet, durch die Desoxypeganin oder eines seiner pharmazeutisch annehmbaren Säureadditionssalze auf kontinuierliche, kontrollierte Weise abgegeben werden.

Arzneiformen, die Wirkstoffe kontrolliert freisetzen, sind im Stand der Technik bekannt. Die Verabreichung pharmazeutisch wirksamer Verbindungen mittels solcher Formulierungen kann oral, transdermal oder anderweitig parenteral erfolgen.

Die im Rahmen der vorliegenden Erfindung geeigneten Formulierungen zur oralen Verabreichung werden nachfolgend beschrieben.

In einer solchen Formulierung ist der pharmazeutische Wirkstoff in einer semipermeablen Membran, z. B. aus Celluloseacetat eingekapselt. Mit einem Bohrer oder einem Laser wird in das Kapselmaterial ein winziges Loch gebohrt. Im Körper des Patienten, der behandelt wird, wird durch das Kapselmaterial Wasser absorbiert. Der pharmazeutische Wirkstoff wird durch osmotischen Druck in der gewünschten, konstanten und kontrollierten Weise allmählich durch die kleine Öffnung getrieben. Solche Systeme sind in den US-Patenten 3,760,805, 3,760,806, 3,760,984, 3,845,770, 3,916,899 und 3,987,790 beschrieben. In diesen Systemen können die pharmazeutischen Wirkstoffe in fester Form oder absorbiert an Ionenaustauscher-Harze vorliegen.

Ein anderes System zur oralen Verabreichung gemäss der vorliegenden Erfindung wird von Sheth und Leeson im US-Patent 4,137,300 beschrieben. Dieses Patent beschreibt eine Formulierung, die eine Wachsmatrix enthält.

Die Wirkstoffe der vorliegenden Erfindung werden mittels entsprechender Formulierungen auf passende und geeignete Weise verabreicht. Die festen Wirkstoffe können in Lösung oder als Dispersion verabreicht werden. Das Lösungs- oder Dispersionsmedium kann anorganisch oder organisch sein. Geeignete Lösungs- oder Suspensionsmedien für Desoxypeganin sind z.B. Wasser, Silikonöl oder Mineralöl.

Unter Silikonölen sollen dabei linearpolymere Dimethylsiloxane und unter Mineralölen die aus mineralischen Rohstoffen (Erdöl, Braun- und Steinkohlenteer, Holz, Torf) gewonnenen Destillationsprodukte, die im wesentlichen aus Gemischen von gesättigten Kohlenwasserstoffen bestehen, verstanden werden.

Um die Verabreichung einer Verbindung mittels einer Formulierung wie vorstehend beschrieben, zu ermöglichen, können dem System folgende Zusatzstoffe zugefügt sein:
Antioxydantien, Synergisten, Stabilisatoren,
Konservierungsmittel,
Geschmackskorrigentien,
Färbemittel,
Lösemittel, Lösungsvermittler,
Tenside (Emulgatoren, Solubilisatoren, Benetzer, Entschäumer),
Viskositäts- und Konsistenzbeeinflusser, Gelbildner,
Resorptionsbeschleuniger,
Adsorptionsmittel,
Feuchthaltemittel,
Gleitmittel (z.B. Fliessregulierungsmittel),
Zerfalls- und Lösungsbeeinflusser,
Füllmittel (Streckmittel),
Peptisatoren,
Freisetzungsverzögerer.

Diese Aufstellung erhebt keinen Anspruch auf Vollständigkeit. Viele Stoffe erfüllen nicht nur eine Funktion, sie sind daher mehreren der angeführten Hilfsstoffgruppen zuzuordnen. So finden z.B. Stärkearten als Füllmittel bei der Tabletten- und Pulverherstellung Verwendung. Sie sind aber zugleich Fliessregulierungsmittel, Adsorptionsmittel, Hydrogelbildner und Viskositätserhöher.

Die in Frage kommenden, physiologisch unbedenklichen Substanzen sind dem Fachmann bekannt.

In einer Formulierung zur transdermalen Verabreichung von Verbindungen gemäss der vorliegenden Erfindung kann der pharmazeutische Wirkstoff in einer Matrix enthalten sein, von der er in der gewünschten allmählichen, konstanten und kontrollierten Weise abgegeben wird. Die Durchlässigkeit der Matrix bei der Freisetzung der Verbindung beruht auf Diffusion. Ein derartiges System ist in dem deutschen Patent DE 33 15 272 beschrieben. Dieses System besteht aus einer undurchlässigen Rückschicht, einem damit verbundenen, besonders aufgebauten übersättigten Wirkstoff-Reservoir aus einer Polymermatrix, einer mit dem Reservoir verbundenen, für den Wirkstoff durchlässigen Haftklebeschicht und einer die Haftklebeschicht abdeckenden, zum Gebrauch wieder ablösbaren Schutzschicht. Auch Systeme, in der die Reservoirschicht so hohe Eigenklebrigkeit aufweist, dass sie gleichzeitig die Haftklebeschicht darstellt, sind möglich.

Wenn der Wirkstoff durch die Haut absorbiert wird, erhält der zu Behandelnde auf diese Weise einen kontrollierten und vorbestimmbaren Zufluss des Wirkstoffes.

Andere geeignete transdermale Formulierungen sind in den US-Patenten 3,742,951, 3,797,494, 3,996,934 und 4,031,894 beschrieben. Diese Formulierungen bestehen grundsätzlich aus einer Rückschicht, die eine der Oberflächen darstellt, einer für den Wirkstoff durchlässigen Klebschicht, die die andere Oberfläche darstellt und letztlich einem Reservoir, das den Wirkstoff zwischen den beiden die Oberflächen bildenden Schichten enthält. Alternativ dazu kann der Wirkstoff auch in einer Vielzahl von Mikrokapseln enthalten sein, die innerhalb der durchlässigen Klebschicht verteilt sind. In jedem Fall wird der Wirkstoff aus dem Reservoir oder den Mikrokapseln durch eine Membran in die für den Wirkstoff durchlässige Klebschicht, die im Kontakt mit der Haut oder Mucosa des zu Behandelnden steht, kontinuierlich abgegeben. Im Falle von Mikrokapseln kann das Kapselmaterial auch als Membran wirken.

Formulierungen, die zur anderweitigen parenteralen Applikation von Desoxypeganin und seinen Salzen in Frage kommen, sind solche, die eine Depotwirkung des Wirkstoffes gestatten. Hierbei wird die Formulierung als Injektionslösung auf nicht-wässriger Grundlage appliziert. Die möglichen Lösungsmittel sind dem Fachmann bekannt. Als Beispiele seien die vegetabilischen Öle erwähnt, die einzelne Pharmakopöen vorschreiben.
Erdnussöl, Olivenöl, Mandelöl, Sonnenblumenöl, Sojabohnenöl und Sesamöl stehen im Vordergrund. Rizinusöl zeigt oftmals eine besonders günstige Löslichkeit für Arzneimittel; daneben sind auch Öle tierischen Ursprungs geeignet. Die Öle sind physiologisch indifferent und gut verträglich. Voraussetzung hierfür ist, dass sie besonders gereinigt sind und niedrige Säure- und Peroxidzahlen aufweisen. Da eine intravenöse Applikation wegen der fehlenden Mischbarkeit mit dem Blutserum nicht möglich ist und zur Lungenembolie führen kann, ist lediglich ihre Anwendung für intramuskuläre und subkutane Injektionspräparate möglich. Ölige Lösungen und Suspensionen verbleiben recht lange am Ort der Applikation (oft bis zu 1 Monat) und geben die Wirkstoffe protrahiert frei.

Die Dosierung des Desoxypeganins oder seiner pharmazeutisch annehmbaren Säureadditionssalze muss so hoch sein, dass eine nachhaltige Wirkung erzielt wird und bedarf einer individuellen Einstellung.
Der Wirkstoffgehalt der jetzigen Formulierung liegt vorzugsweise zwischen 0,1 und 90 Gew.-%, insbesondere bevorzugt zwischen 5 und 20 Gew-%, bezogen auf das Gesamtgewicht der Formulierung.

### Die Erfindung wird durch das folgende Beispiel erläutert:

### Beispiel

Einfluss von Desoxypeganin-HCl auf den freiwilligen Alkoholkonsum von genetisch ethanol-präferenten Ratten.

Versuchstiere waren jeweils 6 weibliche Ratten eines Stammes, der auf die finnische AA-Rattenlinie zurückgeht. Die Tiere dieses Inzuchtstammes bevorzugen 10% Alkohol als Trinkflüssigkeit, wenn ihnen reines Wasser daneben angeboten wird (free choice).

Die Tiere wurden einzeln in Makrolonkäfigen Typ 3 bei 23 °C Raumtemperatur und neunmaligem Luftwechsel pro Stunde gehalten. Nur während der Dunkelphase von 20.00 Uhr bis 08.00 Uhr standen ihnen Trockenfuttergranulat (Altromin 1311; Mehl, dem Vitamine, Mineralstoffe, Aminosäuren und Spurenelemente zugesetzt sind; Hersteller: Altromin Spezialfutterwerke GmbH, Lage), Trinkwasser und Ethanollösung (10% v/v) in unbeschränkten Mengen zur Verfügung, und zwar in besonderen Gefässen, die eine verlustlose Fütterung bzw. Tränkung gewährleisten. Die verbrauchten Mengen wurden gravimetrisch bestimmt, die Trinkmengen automatisch und kontinuierlich mit Hilfe von zwölf Wägezellen. Als Mass der Alkoholpräferenz diente der jeweilige Anteil Ethanollösung an der Gesamttrinkmenge in Prozent: 0% bedeutet, dass nur Wasser, keine Ethanollösung getrunken wurde, 100% bedeutet, dass ausschliesslich Ethanollösung getrunken wurde. Die durchschnittliche Alkoholpräferenz der unbehandelten Versuchstiere betrug 78% bzw. 83%.

Die Ergebnisse sind in der folgenden Tabelle wiedergegeben:

**Tabelle:**

| Einfluss von Desoxypeganin-HCl auf das ingestive Verhalten von weiblichen ethanol-präferenten Ratten (n=6). Die während der jeweils dreitägigen Vorperiode ermittelten Werte stehen in runden Klammern. | | |
|---|---|---|
| Dosis [mg/kg oral] | 15 | 30 |
| Körpergewicht, KG [g] | 215 ± 6,2 | 213 ± 4,9 |
| Alkoholpräferenz [%] | (82,8 ± 6,4) | (77,8 ± 4,3) |
| | 45,0 ± 12,9** | 51,7 ± 6,4*** |
| Alkoholkonsum, absolut [g/kg KG] | (6,47 ± 0,43) | (6,30 ± 0,34) |
| | 3,17 ± 0,89** | 3,71 ± 0,42 *** |
| Gesamttrinkmenge [g/kg KG] | (97,2 ± 2,3) | (100,9 ± 1,9) |
| | 89,8 ± 5,6 ns | 90,6 ± 6,1,ns |
| Nahrungsaufnahme [g/kg KG] | (55,2 ± 1,3) | (55,7 ± 2,1) |
| | 57,7 ± 2,6 ns | 44,6 ± 7,6 ns |
| Student's t-Test für gepaarte Werte | | |

| | | |
|---|---|---|
| ** p < 0,01 | | |
| *** p < 0,001 ns = nicht signifikant | | |

Wie die Tabelle zeigt, lässt sich durch Verabreichung von Desoxypeganin das Verlangen nach Alkohol beträchtlich reduzieren. Nahrungsaufnahme und Gesamttrinkmenge werden nicht signifikant verändert.

## Patentansprüche

1. Verwendung von Desoxypeganin und / oder eines seiner pharmazeutisch verträglichen Säureadditionssalze zur Herstellung eines Arzneimittels zur Behandlung des chronischen Alkoholismus.

2. Verwendung nach Anspruch 1 für eine transdermale, orale oder parenterale Applikation.

3. Verwendung nach Anspruch 1 oder 2 wobei das Arzneimittel 0,1 bis 90 Gew.-%, vorzugsweise 5 bis 20 Gew.-% seines Gesamtgewichtes an Wirkstoff sowie übliche Zusatzstoffe enthält.

4. Verwendung nach einem der Ansprüche 1 bis 3, wobei das Arzneimittel eine Depotwirkung aufweist.

5. Verwendung nach einem der Ansprüche 1 bis 4, wobei der Wirkstoff in Form einer Lösung oder als Dispersion in einem anorganischen oder organischen Medium vorliegt.

6. Pharmazeutische Formulierung zur Behandlung des chronischen Alkoholismus enthaltend Desoxypeganin und / oder eines seiner pharmazeutisch verträglichen Säureadditionssalze, **dadurch gekennzeichnet, dass** der Wirkstoff in ein transdermales therapeutisches System eingebracht ist, enthaltend eine undurchlässige Rückschicht, ein Wirkstoffreservoir, gegebenenfalls einer die Abgabe des Wirkstoffs steuernden Membran, einer Haftktebeeinrichtung zur Befestigung des Systems auf der Haut und gegebenenfalls einer vor der Applizierung des Systems ablösbaren Schutzschicht.

## Claims

1. The use of deoxypeganine and/or one of its pharmaceutically tolerable acid addition salts for producing a medicament for the treatment of chronic alcoholism.

2. The use as claimed in claim 1 for transdermal, oral or parenteral administration.

3. The use as claimed in claim 1 or 2, where the medicament comprises 0.1 to 90% by weight, preferably 5 to 20% by weight, of its total weight of active substance, and customary additives.

4. The use as claimed in one of claims 1 to 3, where the medicament has a depot action.

5. The use as claimed in one of claims 1 to 4, where the active substance is present in the form of a solution or as a dispersion in an inorganic or organic medium.

6. A pharmaceutical formulation for the treatment of chronic alcoholism containing deoxypeganine and/or one of its pharmaceutically tolerable acid addition salts, **characterized in that** the active substance is incorporated into a transdermal therapeutic system, comprising an impermeable back layer, an active-substance reservoir, if appropriate a membrane controlling the release of the active substance, a pressure-sensitive adhesive device for fixing,the system to the skin and, if appropriate, a protective layer which can be removed before application of the system.

## Revendications

1. Utilisation de la désoxypéganine et/ou d'un de ses sels d'addition d'acide pharmaceutiquement acceptables pour la préparation d'un médicament destiné au traitement de l'alcoolisme chronique.

2. Utilisation selon la revendication 1 pour une application par voie transdermique, orale ou parentérale.

3. Utilisation selon la revendication 1 ou 2, le médicament contenant 0,1 à 90% en poids, de préférence 5 à 20% en poids de son poids total en substance active ainsi que des additifs usuels.

4. Utilisation selon l'une quelconque des revendications 1 à 3, le médicament présentant un effet retard.

5. Utilisation selon l'une quelconque des revendications 1 à 4, la substance active se trouvant sous forme d'une solution ou d'une dispersion dans un milieu inorganique ou organique.

6. Formulation pharmaceutique destinée au traitement de l'alcoolisme chronique, contenant de la désoxypéganine et/ou un de ses sels d'addition d'acide pharmaceutiquement acceptables, **caractérisée en ce que** la substance active est introduite dans un système thérapeutique transdermique contenant une couche arrière imperméable, un réservoir de substance active, le cas échéant une membrane contrôlant la libération de la substance active, un dispositif auto-adhésif destiné à la fixation du système sur la peau et le cas échéant une couche de protection pouvant être enlevée avant l'application du système.
